(19) Europäisches Patentamt
European Patent Office
Office européen des brevets

(11) Veröffentlichungsnummer: **0 198 829**
**B1**

(12) **EUROPÄISCHE PATENTSCHRIFT**

(45) Veröffentlichungstag der Patentschrift: **18.04.90**

(51) Int. Cl.$^5$: **C 07 C 405/00**

(21) Anmeldenummer: **85900066.3**

(22) Anmeldetag: **21.12.84**

(86) Internationale Anmeldenummer:
**PCT/DE84/00280**

(87) Internationale Veröffentlichungsnummer:
**WO 85/02841 04.07.85 Gazette 85/15**

(54) **9-HALOGEN-PROSTAGLANDINDERIVATE, VERFAHREN ZU IHRER HERSTELLUNG UND IHRE VERWENDUNG ALS ARZNEIMITTEL.**

(30) Priorität: **22.12.83 DE 3347128**

(43) Veröffentlichungstag der Anmeldung:
**29.10.86 Patentblatt 86/44**

(45) Bekanntmachung des Hinweises auf die Patenterteilung:
**18.04.90 Patentblatt 90/16**

(84) Benannte Vertragsstaaten:
**AT BE CH DE FR GB LI LU NL SE**

(56) Entgegenhaltungen:
**DE-A-2 950 027**
**DE-A-3 126 924**
**LU-A- 71 563**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **SKUBALLA, Werner**
**Olwenstr. 25**
**D-1000 Berlin 28 (DE)**
Erfinder: **RADÜCHEL, Bernd**
**Gollanczstr. 132**
**D-1000 Berlin 28 (DE)**
Erfinder: **VORBRÜGGEN, Helmut**
**Wilkestr. 7**
**D-1000 Berlin 27 (DE)**
Erfinder: **ELGER, Walter**
**Schorlemer Allee 12b**
**D-1000 Berlin 33 (DE)**
Erfinder: **LOGE, Olaf**
**Bekassinenweg 37**
**D-1000 Berlin 27 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue 9-Halogen-$\Delta^2$-prostaglandinderivate, Verfahren zu ihrer Herstellung sowie ihre Verwendung als Arzneimittel.

Aus dem sehr umfangreichen Stand der Technik der Prostaglandine und ihrer Analoga weiß man, daß diese Stoffklasse aufgrund ihrer biologischen und pharmakologischen Eigenschaften zur Behandlung von Säugetieren, einschließlich des Menschen, geeignet ist. Ihre Verwendung als Arzneimittel stößt jedoch häufig auf Schwierigkeiten. Die meisten natürlichen Prostaglandine besitzen eine für therapeutische Zwecke zu kurze Wirkungsdauer, da sie zu rasch durch verschiedene enzymatische Prozesse metalolisch abgebaut werden. Alle Strukturveränderungen haben das Ziel, die Wirkungsdauer sowie die Selektivität der Wirksamkeit zu steigern.

Durch die deutschen Offenlegungsschriften 29 50 027 und 31 26 924 sind Prostanderivate mit einem Chlor- oder Fluoratom in 9-Stellung bekannt. Es wurde nun gefunden, daß durch Einführung einer Doppelbindung in die 2,3-Position der 9-Halogen-prostaglandine eine längere Wirkungsdauer, größere Selektivität und bessere Wirksamkeit erzielt werden kann.

Die Erfindung betrifft 9-Halogen-$\Delta^2$-prostanderivate der allgemeinen Formel I

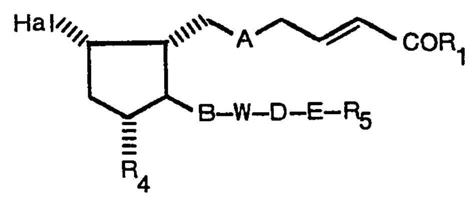

(I),

worin

Hal ein α- oder β-ständiges Fluor- oder Chloratom sein kann,

$R_1$ den Rest $OR_2$ mit $R_2$ in der Bedeutung eines Wasserstoffatoms, eines $C_{1-10}$-Alkyls, $C_{3-10}$-Cycloalkyls, Phenyls oder eines 5- oder 6-gliedrigen heterocyclischen Restes oder $R_1$ den Rest $NHR_3$ mit $R_3$ in der Bedeutung eines organischen Carbonsäure- oder Sulfonsäurerestes mit 1—15 C-Atomen oder des Restes $R_2$ und

A eine $—CH_2—CH_2—$ oder cis-CH=CH-Gruppe,

B eine $—CH_2—CH_2—$, oder trans-CH=CH— oder eine —C≡C-Gruppe,

W eine

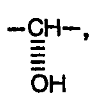 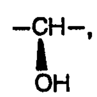 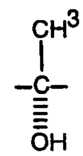 or 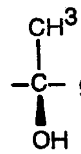 group,

in denen die OH-Gruppen durch Tetrahydropyranyloxy, Tetrahydrofuranyloxy, α-Ethoxyethoxy, Trimethylsilyloxy, Dimethyl-Tert.-butylsilyloxy, Acetoxy, Propionyloxy, Butyryloxy oder Benzoyloxy ersetzt werden können,

D und E gemeinsam eine direkte Bindung oder

D eine gerad- oder verzweigtkettige gesättigte Alkylengruppe mit 1—5 C-Atomen, die gegebenenfalls durch Fluoratome substituiert sein kann,

E ein Sauerstoff- oder Schwefelatom, eine direkte Bindung, eine —C≡C-Bindung oder eine $—CR_6=CR_7$-Gruppe darstellt, wobei $R_6$ ein Wasserstoffatom oder eine Alkylgruppe mit 1—6 C-Atomen und $R_7$ ein Wasserstoffatom, eine Alkylgruppe mit 1—6 C-Atomen oder ein Halogenatom bedeuten,

$R_4$ eine freie Hydroxygruppe, die durch Tetrahydropyranyloxy, Tetrahydrofuranyloxy, α-Ethoxyethoxy, Trimethylsilyloxy, Dimethyl-tert.-butylsilyloxy, Acetoxy, Propionyloxy, Butyryloxy oder Benzoyloxy ersetzt werden kann.

$R_5$ ein Wasserstoffatom, eine $C_{1-6}$-Alkyl-, eine Halogen-substituierte $C_{1-6}$-Alkyl-, eine $C_{1-6}$-Cycloalkyl-, eine Phenyl- oder eine 5- oder 6-gliedrige heterocyclische Gruppe und, falls $R_2$ die Bedeutung eines Wasserstoffatoms hat, deren Salze mit physiologisch verträglichen Basen bedeuten.

Als Alkylgruppen $R_2$ sind gerade oder verzweigte Alkylgruppen mit 1—10 C-Atomen zu betrachten, wie beispielsweise Methyl, Äthyl, Propyl, Butyl, Isobutyl, tert.-Butyl, Pentyl, Neopentyl, Hexyl, Heptyl, Decyl. Als bevorzugte Alkylgruppen $R_2$ sind solche mit 1—4 C-Atomen, wie z.B. Methyl, Äthyl, Propyl, Isobutyl, Butyl zu nennen.

Die Cycloalkylgruppe $R_2$ kann im Ring 3—10, vorzugsweise 5 und 6 Kohlenstoffatome enthalten. Beispielsweise seien genannt Cyclopentyl-, Cyclohexyl.

Als heterocyclische Gruppen $R_2$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, 2-Tetrazolyl u.a.

Als Säurerest $R_3$ kommen physiologisch verträgliche Säurereste in Frage. Bevorzugte Säuren sind organische Carbonsäuren und Sulfonsäuren mit 1—15 Kohlenstoffatomen, die der aliphatischen, cycloaliphatischen, aromatischen, aromatisch-aliphatischen und heterocyclischen Reihe angehören. Diese Säuren können gesättigte, ungesättigte und/oder mehrbasisch und/oder in üblicher Weise substituiert sein. Als Beispiele für die Substituenten seien Alkyl-, Hydroxy-, Alkoxy-, Oxo- oder Aminogruppen oder Halogenatome erwähnt. Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diäthylessigsäure, tert.-Butylessigsäure, Cyclopropylessigsäure, Cyclopentylessigsäure, Cyclohexylessigsäure, Cyclopropancarbonsäure, Cyclohexancarbonsäure, Phenylessigsäure, Phenoxyessigsäure, Methoxyessigsäure, Äthoxyessigsäure, Mono-, Di- und Trichloroessigsäure, Aminoessigsäure, Diäthylaminoessigsäure, Piperidinoessigsäure, Morpholinoessigsäure, Milchsäure, Bernsteinsäure, Adipinsäure, Benzoesäure, mit Halogen-, Trifluormethyl-, Hydroxy-, Alkoxy- oder Carboxy-Gruppen substituierte Benzoesäuren, Nikotinsäure, Isonikotinsäure, Furan-2-carbonsäure, Cyclopentylpropionsäure. Als besonders bevorzugte Acylreste werden solche mit bis zu 10 Kohlenstoffatomen betrachtet. Als Sulfonsäuren kommen beispielsweise Methansulfonsäure, Äthansulfonsäure, Isopropansulfonsäure, β-Chloräthansulfonsäure, Butansulfonsäure, Cyclopentansulfonsäure, Cyclohexansulfonsäure, Benzolsulfonsäure, p-Toluolsulfonsäure, p-Chlorbenzolsulfonsäure, N,N-Dimethylaminosulfonsäure, N,N-Disäurethylaminosulfonsäure, N,N-Bis-(β-chloräthyl)-aminosulfonsäure, N,N-Diisobutylaminosulfonsäure, N,N-Dibutylaminosulfonsäure, Pyrrolidino-, Piperidino-, Piperazino-, N-Methylpiperazino- und Morpholinosulfonsäure in Frage.

Die Hydroxygruppen in W und $R_4$ können funktionell abgewandelt sein, beispielsweise durch Verätherung oder Veresterung, wobei auch die abgewandelte Hydroxygruppe in W α- oder β-ständit sein kann.

Als Äther- und Acylreste konnen die dem Fachmann bekannten Reste in Betracht. Bevorzugt sind leicht abspaltbare Ätherreste, wie beispielsweise der Tetrahydropyranyl-, Tetrahydrofuranyl-, α-Äthoxyäthyl-, Trimethylsilyl-, Dimethyl tert.-butyl-silyl und Tribenzyl-silylrest.

Als Acylreste kommen die gleichen wie für $R_3$ genannt in Frage, namentlich genannt seien beispielsweise Acetyl, Propionyl, Butyryl und Benzoyl.

Als Alkylgruppen $R_5$ kommen gerad- und verzweigtkettige, gesättigte Alkylreste mit 1—6 C-Atomen in Frage. Beispielsweise genannt seien Methyl-, Äthyl-, Propyl-, Butyl-, Isobutyl- tert.-Butyl-, Pentyl-, Hexyl-.

Sind die Alkylgruppen $R_5$ Halogen-substituiert, kommen als Halogene Fluor, Chlor und Brom in Frage.

Die Cycloalkylgruppe $R_5$ kann im Ring 3—6 Kohlenstoffatome enthalten. Beispielsweise genannt seien Cyclopropyl, Cyclobutyl, Cyclopentyl, Cyclohexyl.

Als heterocyclische Gruppen $R_5$ kommen 5- und 6-gliedrige Heterocyclen in Frage, die wenigstens 1 Heteroatom, vorzugsweise Stickstoff, Sauerstoff oder Schwefel enthalten. Beispielsweise seien genannt 2-Furyl, 2-Thienyl, 2-Pyridyl, 3-Pyridyl, 4-Pyridyl, Oxazolyl, Thiazolyl, Pyrimidinyl, Pyridazinyl, Pyrazinyl, 3-Furyl, 3-Thienyl, u.a.

Als Alkylengruppe D kommen geradkettige oder verzweigtkettige, gesättigte Alkylenreste mit 1—5 C-Atomen in Frage, die gegebenenfalls durch Fluoratome substituiert sein können. Beispielsweise seien genannt: Methylen, Fluormethylen, Difluormethylen, Äthylen, 1,2-Propylen, Äthyläthylen, Trimethylen, Tetramethylen, Pentamethylen, 1,1-Difluoräthylen, 1-Fluoräthylen, 1-Methyltetramethylen, 1-Methyl-trimethylen, 1-Methylen-äthylen, 1-Methylen-tetramethylen.

Als Alkylgruppen $R_6$ und $R_7$ kommen geradkettige oder verzweigtkettige Alkylgruppen mit 1—6 C-Atomen, vorzugsweise 1—4 C-Atomen, wie sie bereits für die Alkylgruppen $R_5$ genannt wurden, in Betracht. Unter Halogen für $R_7$ werden die Halogenatome Fluor, Chlor und Brom verstanden, wobei Chlor bevorzugt sein soll.

Zur Salzbildung sind anorganische und organische Basen geeignet, wie sie dem Fachmannn zur Bildung physiologisch verträglicher Salze bekannt sind. Beispielsweise seien genannt Alkalihydroxide, wie Natrium- und Kaliumhydroxid, Erdalkalihydroxide, wie Calciumhydroxid, Ammoniak, Amine, wie Äthanolamin, Diäthanolamin, Triäthanolamin, N-Methylglucamin, Morpholin, Tris-(hydroxymethyl)-methylamin usw.

Die Erfindung betrifft außerdem ein Verfahren zur Herstellung der erfindungsgemäßen 9-Halogen-$\Delta^2$-prostanderivate der allgemeinen Formel I, dadurch gekennzeichnet, daß man in an sich bekannter Weise in eine Verbindung der allgemeinen Formel II,

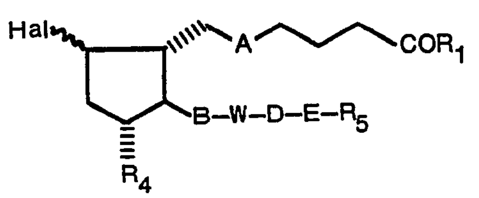

(II),

eine Doppelbindung in 2-Stellung einführt, indem man mit einem Lithiumdialkylamid der allgemeinen Formel III,

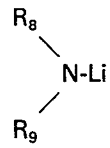
(III),

worin $R_8$ und $R_9$ eine Alkylgruppe mit 1—10 Kohlenstoffatomen oder eine Cycloalkylgruppe mit 3—6 Kohlenstoffatomen oder Trialkylsilylgruppe mit Alkyl in der Bedeutung $C_1$—$C_4$-Alkyl darstellen, umsetzt, anschließend mit einer Phenylverbindung der allgemeinen Formel IV,

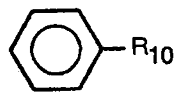
(IV),

worin $R_{10}$ die Reste

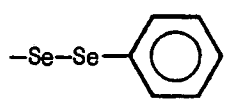
,
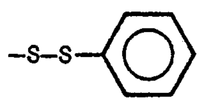
oder SeBr

oder Se≡r darstellt, behandelt und anschließend in beliebiger Reihenfolge eine oxidative Eliminierung durchführt und/oder gegebenenfalls Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

Als Alkylgruppen $R_8$ und $R_9$ kommen alle die für $R_2$ genannten Gruppen in Betracht. Es sollen auch dieselben Gruppen bevorzugt sein. Als Cycloalkylgruppen $R_8$ und $R_9$ mit 3—6 C-Atomen sind gemeint: Cyclopropyl, Cyclobutyl, Cyclopentyl, Cyclohexyl.

Die Umsetzung der Verbindungen der allgemeinen Formel II mit einer Lithiumamidverbindung der allgemeinen Formel III, vorzugsweise Lithiumdiisopropylamid, erfolgt in an sich bekannter Weise in einem inerten Lösungsmittel oder Lösungsmittelgemisch wie z.B. Tetrahydrofuran, Diäthyläther, Hexamethylphosphorsäureamid, Dimethoxyäthan usw., vorzugsweise Tetrahydrofuran. Die Reaktion wird bei Temperaturen zwischen −100° und 0°C, vorzugsweise −80°C bis −50°C, durchgeführt. Die Umsetzung mit Diphenyldiselenid, Diphenyldisulfid oder Phenylselenylbromid erfolgt bei Temperaturen zwischen −100°C und +30°C, vorzugsweise −70°C und 0°C. Die Oxidation der erhaltenen Selenverbindung erfolgt mit Wasserstoffperoxid in einem inerten Lösungsmittel oder Lösungsmittelgemisch wie z.B. Tetrahydrofuran, Essigsäureäthylester, Essigsäuremethylester, Methanol, Äthanol, Isopropanol usw. bei Temperaturen zwischen −20°C und +60°C, vorzugsweise 0°C—30°C, wobei bereits die intermediäre Selenoxidverbindung unter Bildung der $\Delta^2$-Doppelbindung zerfällt.

Die Oxidation der erhaltenen Schwefelverbindung wird zweckmässigerweise mit Natriumperjodat in Gegenwart eines Alkohols, wie beispielsweise Methanol, Äthanol unter Zusatz von Wasser bei Temperaturen zwischen 0°C und 60°C, vorzugsweise 10°C bis 30°C durchgeführt. Der Zerfall des gebildeten Sulfoxids in die $\Delta^2$-Verbindung der allgemeinen Formel I erfolgt bei Temperaturen zwischen 30°C und 120°C unter Zusatz einer geringen Menge Calciumcarbonat in einem inerten Lösungsmittel wie beispielsweise Toluol, Xylol, Tetrachlorkohlenstoff usw.

Die Verseifung der Prostaglandinester wird nach den dem Fachmann bekannten Methoden durchgeführt, wie beispielsweise mit basischen Katalysatoren oder enzymatisch mit beispielsweise Lipase, Schweineleberesterase oder Bäckerhefe.

Die Erführung der Estergruppe —$OR_2$ für $R_1$, bei welcher $R_2$ eine Alkylgruppe mit 1—10 C-Atomen darstellt, erfolgt nach den dem Fachmann bekannten Methoden. Die Carboxyverbindungen werden beispielsweise mit Diazokohlenwasserstoffen in an sich bekannter Weise umgesetzt. Die Veresterung mit Diazokohlenwasserstoffen erfolgt zum Beispiel dadurch, daß man eine Lösung des Diazokohlenwasserstoffes in einem inerten Lösungsmittel, vorzugsweise in Diäthyläther mit der Carboxyverbindung in dem gleichen oder in einem anderen inerten Lösungsmittel, wie zum Beispiel Methylenchlorid, vermischt. Nach beendeter Umsetzung in 1 bis 30 Minuten wird das Lösungsmittel entfernt und der Ester in üblicher Weise gereinigt. Diazoalkane sind entweder bekannt oder können nach bekannten Methoden hergestellt werden [Org.Reactions Bd. *8*, Seiten 389—394 (1954)].

Die Einführung der Estergruppe —$OR_2$ für $R_1$, bei welcher $R_2$ eine substituierte oder unsubstituierte Arylgruppe darstellt, erfolgt nach den dem Fachmann bekannten Methoden. Beispielsweise werden die Carboxyverbindungen mit den entsprechenden Arylhydroxyverbindungen mit Dicyclohexylcarbodiimid in Gegenwart einer geeigneten Base, beispielsweise Pyridin oder Triäthylamin, in einem inerten Lösungsmittel umgesetzt. Als Lösungsmittel kommen Methylenchlorid, Äthylenchlorid, Chloroform, Essigester,

Tetrahydrofuran, vorzugsweise Chloroform, in Frage, Die Reaktion wird bei Temperaturen zwischen −30°C und +50°C, vorzugsweise bei +10°C, durchgeführt.

Die Prostaglandin-Derivate der allgemeinen Formel I mit $R_1$ in der Bedeutung einer Hydroxygruppe können mit geeigneten Mengen der entsprechenden anorganischen Basen unter Neutralisierung in Salze überführt werden. Beispielsweise erhält man beim Lösen der entsprechenden PG-Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Die Herstellung der Aminsalze erfolgt in üblicher Weise. Dazu wird die PG-Säure zum Beispiel in einem geeigneten Lösungsmittel, wie Äthanol, Aceton, Diäthyläther oder Benzol gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Die funktionelle Abwandlung der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluolsulfonsäure, umgesetzt. Das Dihydropyran wird im Überschuß angewandt, vorzugsweise in der 4- bis 10-fachen Menge des theoretischen Bedarfs. Die Umsetzung ist normaleweise bei 0°C—30°C nach 15—30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer funktionell abgewandelten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmäßigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20°C und 80°C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0°C und 80°C durchgeführt.

Die Verseifung der Acylgruppen erfolgt beispielsweise mit Alkali- oder Erdalkali-carbonaten oder -hydroxyden in einem Alkohol oder der wäßrigen Lösung eines Alkohols. Als Alkohole kommen aliphatische Alkohole in Betracht, wie zum Beispiel Methanol, Äthanol, Butanol usw., vorzugsweise Methanol. Als Alkalicarbonate und -hydroxyde seien Kalium- und Natriumsalze genannt, bevorzugt sind jedoch die Kaliumsalze. Als Erdalkalicarbonate und -hydroxyde sind beispielsweise geeignet Calciumcarbonat, Calciumhydroxyd und Bariumcarbonat. Die Umsetzung erfolgt bei −10°C bis 70°C, vorzugsweise bei 25°C.

Die Einführung der Amidgruppe $NHR_3$ für $R_1$ erfolgt nach den dem Fachmann bekannten Methoden. Die Carbonsäuren den allgemeinen Formel I ($R_2$=H) werden zunächst in Gegenwart eines tertiären Amins, wie beispielsweise Triäthylamin, mit Chlorameisensäureisobutylester in das gemischte Anhydrid überführt. Die Umsetzung des gemischten Anhydrids mit dem Alkalisalz des entsprechenden Amids oder mit Ammoniak ($R_3$= H) erfolgt in einem inerten Lösungsmittel oder Lösungsmittelgemisch, wie beispielsweise Tetrahydrofuran, Dimethoxyäthan, Dimethylformamid, Hexamethylphosphorsäuretriamid, bei Temperaturen zwischen −30°C und +60°C, vorzugsweise bei 0°C bis 30°C.

Eine weitere Möglichkeit für die Einführung der Amidgruppe $NHR_3$ für $R_1$ besteht in der Umsetzung einer 1-Carbonsäure der allgemeinen Formel I ($R_2$=H), in der freie Hydroxygruppen gegebenenfalls intermediär geschützt sind, mit Verbindungen der allgemeinen Formel V,

$$O{=}C{=}N{-}R_3 \qquad V,$$

worin $R_3$ die obenangegebene Bedeutung hat.

Die Umsetzung der Verbindung der allgemeinen Formel I ($R_2$=H) mit einem Isocyanat der allgemeinen Formel V erfolgt gegebenenfalls unter Zusatz eines tertiären Amins, wie z.B. Triäthylamin oder Pyridin. Die Umsetzung kann ohne Lösungsmittel oder in einem inerten Lösungsmittel, vorzugsweise Acetonitril, Tetrahydrofuran, Aceton, Dimethylacetamid, Methylenchlorid, Diäthyläther, Toluol, bei Temperaturen zwischen −80°C bis 100°C, vorzugsweise bei 0°C bis 30°C, vorgenommen werden.

Enthält das Ausgangsprodukt CH-Gruppen im Prostanrest, so werden diese CH-Gruppen auch zur Reaktion gebracht. Werden letztlich Endprodukte gewünscht, die freie Hydroxylgruppen im Prostanrest enthalten, geht man zweckmäßigerweise von Ausgangsprodukten aus, in denen diese durch vorzugsweise leicht abspaltbare Äther- oder Acylreste intermediär geschützt sind.

Die als Ausgangsmaterial dienenden Verbindungen der allgemeinen Formel II mit Hal in der Bedeutung eines Fluoratoms können beispielsweise gemäß der deutschen Offenlegungsschrift 31 26 924

hergestellt werden, indem man in an sich bekannter Weise eine Verbindung der allgemeinen Formel VI,

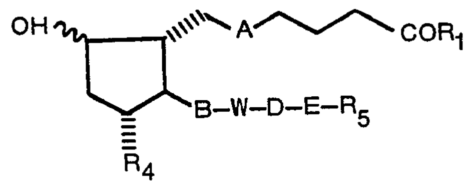

(VI),

worin die OH-Gruppe α- oder β-ständig sein kann, $R_1$, $R_4$, $R_5$, A, B, W, D und $R_5$ die oben angegebene Bedeutung haben und freie OH-Gruppen in $R_4$ und W gegebenenfalls geschützt sind, über einen intermediären Sulfonsäureester mit einem Tetraalkylammoniumfluorid umsetzt.

Die als Ausgangsmaterial dienenden Verbindungen der allgemeinen Formel II mit Hal in der Bedeutung eines Chloratoms können beispielsweise gemäß der deutschen Offenlegungsschrift 29 50 027 hergestellt werden, indem man in an sich bekannter Weise eine Verbindung der allgemeinen Formel VI a) über einen intermediären Sulfonsäurester mit einem Chlorid der allgemeinen Formel VII,

$$R_{11}Cl \qquad (VII),$$

worin $R_{11}$ die Bedeutung Lithium, Natrium, Kalium oder Tetraalkylammonium mit Alkyl als gesättigten $C_1$—$C_6$-Rest hat, umsetzt oder b) mit dem Reagenz Tetrachlorkohlenstoff bzw. Hexachloräthan/Triphenylphosphin chloriert.

Im Vergleich zu PGE-Derivaten zeichnen sich die neuen 9-Halogen-$\Delta^2$-prostaglandine durch größere Stabilität aus.

Die neuen 9-Halogen-$\Delta^2$-prostanderivate der allgemeinen Formel I sind wertvolle Pharmaka, da sie bei ähnlichem Wirkungsspektrum eine wesentlich verbesserte (höhere Spezifität) und vor allem wesentlichen längere Wirkung aufweisen als die entsprechenden natürlichen Prostaglandine.

Die neuen prostaglandin-Analoga wirken stark uterotrop und luteolytisch, d. h. zur Auslösung einer Uteruskontraktion bzw. Luteolyse benötigt man wesentlich geringere Dosierungen als bei den entsprechenden natürlichen prostaglandinen.

Auch zur Auslösung von Aborten, insbesondere nach oraler oder intravaginaler Applikation, sind wesentlich geringere Mengen der neuen Prostaglandinanaloga im Vergleich zu den natürlichen Prostaglandinen erforderlich.

Bei der Registrierung der isotonischen Unteruskontraktion an der narkotisierten Ratte und am isolierten Rattenuterus zeigt sich, dass die erfindungsgemässen Substanzen wesentlich wirksamer sind und ihre Wirkungen länger anhalten als bei den natürlichen Prostaglandinen.

Die neuen Prostaglandinderivate sind geeignet, nach einmaliger enteraler oder parenteraler Applikation eine Menstruation zu induzieren oder eine Schwangerschaft zu unterbrechen. Sie eignen sich ferner zur Synchronisation des Sexualzyklus bei weiblichen Säugetieren wie Kaninchen, Rindern, Pferden, Schweinen usw. Ferner eignen sich die erfindungsgemässen Prostaglandin-Derivate zur Cervixdilatation als Vorbereitung für diagnostische oder therapeutische Eingriffe.

Die gute Gewebsspezifität der erfindungsgemässen antifertil wirksamen Substanzen zeigt sich bei der Untersuchung an anderen glattmuskulären Organen, wie beispielsweise am Meerschweinchen-Ileum oder an der isolierten Kaninchen-Trachea, wo eine wesentlich geringere Stimulierung zu beobachten ist als durch die natürlichen Prostaglandine. Die erfindungsgemässen Substanzen wirken auch bronchospasmolytisch. Ausserdem bewirken sie eine Abschwellung der Nasenschleimhaut.

Die erfindungsgemässen Wirkstoffe hemmen die Magensäuresekretion, zeigen einen zytoprotektiven und ulcusheilenden Effekt und wirken damit den unerwünschten Folgen nichtsteroidaler Entzündungshemmstoffe (Prostaglandin-synthese — Inhibitoren) entgegen. Sie werken ausserdem an der Leber und auch an der Bauchspeicheldrüse zytoprotektiv.

Einige der Verbindungen wirken blutdrucksenkend, regulierand bei Herzrhythmusstörungen und hemmend auf die Plättchenaggregation mit den sich daraus ergebenden Einsatzmöglichkeiten. Die neuen Prostaglandine können auch in Kombination z.B. mit β-Blockern und Diuretika verwendet werden.

Für die medizinische Anwendung können die Wirkstoffe in eine für die Inhalation, für orale, parenterale oder lokale (z.B. vaginale) Applikation geeignete Form überführt werden.

Zur Inhalation werden zweckmässigerweise Ärosollösungen hergestellt.

Für die orale Applikation sind beispielsweise Tabletten, Dragees oder Kapseln geeignet.

Für die parenterale Verabreichung werden sterile, injizierbare, wässrige oder ölige Lösungen benutzt.

Für die vaginale Applikation sind z.B. Zäpfchen geeignet und üblich.

Die Erfindung betrifft damit auch Arzneimittel auf Basis der Verbindungen der allgemeinen Formel I und der üblichen Hilfs- und Trägerstoffe.

Die erfindungsgemässen Wirkstoffe sollen in Verbindung mit den in der Galenik bekannten und üblichen Hilfsstoffen, z.B. zur Herstellung von Präparaten zur Auslösung eines Abortes, zur Zyklussteuerung, zur Einleitung einer Geburt oder zur Behandlung der Hypertonie dienen. Für diesen Zweck aber

auch für die übrigen Anwendung können die Präparate 0,01—50 mg der aktiven Verbindung enthalten.

Die folgenden Beispiele sollen die Erfindung näher erläutern, ohne dass damit eine Begrenzung vorgenommen wird.

### Beispiel 1

(2E,13E)-(9R,11R,15R)-11,15-Dihydroxy-9-fluor-16-phenoxy-17,18,19,20-tetranor-2,13-prostadiensäuremethylester

Zu einer Lösung von 0,28 ml Diispropylamin in 2,1 ml abs. Tetrahydrofuran tropft man bei −20°C 0,8 ml einer 1,6 molaren Butyllithiumlösung in Hexan und rührt 30 Minuten bei −20°C unter Argon. In diese Lösung tropft man bei −70°C eine Lösung von 370 mg (13E)-(9R,11R,15R)-11,15-Bis-(tetrahydropyran-2-yloxy)-9-fluor-16-phenoxy-17,18,19,20-tetranor-13-prostensäuremethylester in 3,3 ml abs. Tetrahydrofuran, rührt 30 Minuten bei −70°C und tropft anschließend bei −70°C eine Lösung von 334 mg Diphenyldiselenid in 2,1 ml abs. Tetrahydrofuran zu. Man rührt die Reaktionsmischung 60 Minuten bei −40°C, versetzt mit Wasser, säuert mit 3%iger Schwefelsäure auf pH6 an, extrahiert dreimal mit Äther, wäscht mit Wasser neutral, trocknet über Magnesiumsulfat und dampft im Vakuum ein. Den Rückstand filtriert man mit Hexan/Essigester (85+15) über Kieselgel. Dabei erhält man 285 mg der 2-Phenylselenverbindung als gelbliches Öl.

Zur Oxidation versetzt man eine Lösung von 285 mg der vorstehend hergestellten Selenverbindung in 6 ml Methylenchlorid und 0,15 ml Pyridin mit 0,15 ml 30%iger Wasserstoffperoxidlösung und rührt 1,5 Stunden bei 25°C Anschließend verdünnt man mit Methylenchlorid, schüttelt mit 5%iger Natriumbicarbonatlösung, wäscht mit Wasser neutral, trocknet über Magnesiumsulfat und dampft im Vakuum ein. Den Rückstand filtriert man mit Toluol/Essigester (9+1) über Kieselgel. Dabei erhält man 160 mg (2E,13E)-(9R,11R,15R)-11,15-Bis-(tetrahydropyranyloxy)-9-fluor-16-phenoxy-17,18,19,20-tetranor-2,13-prostadiensäuremethylester.

IR ($CHCl_3$): 2943, 2860, 1716, 1659, 1600, 1578, 1495, 972/cm.

Zur Schutzgruppenabspaltung rührt man 160 mg der vorstehend hergestellten$\Delta^2$-Verbindung 16 Stunden bei 25°C mit 8 ml einer Mischung aus Essigsäure, Wasser, THF (65+35+10) und dampf anschließend im Vakuum ein. Den Rückstand reinigt man durch Chromatographie an Kieselgel. Mit Methylenchlorid/Aceton (8+2) erhält man 102 mg der Titelverbindung als farbloses Öl.

IR: 3590, 3420 (breit), 2950, 2860, 1712, 1657, 1599, 1588, 1495, 970/cm.

Das Ausgangsmaterial für die obige Titelverbindung wird wie folgt hergestellt:

1 a)

(13E)-(9R,11R,15R)-11,15-Bis-(tetrahydropyran-2-yloxy)-9-fluor-16-phenoxy-17,18,19,20-tetranor-13-prostensäuremethylester

Zu einer Lösung von 250 mg (13E)-(9R,11R,15R)-11,15-Dihydroxy-9-fluor-16-phenoxy-17,18,19,20-tetranor-13-prostensäuremethylester und 160 mg Dihydropyran in 16 ml Methylenchlorid fügt man bei 0°C 2,5 mg p-Toluolsulfonsäure und rührt 30 Minuten bei 0°C. Anschließend verdünnt man mit Äther, schüttelt mit 5%iger Natriumbicarbonatlösung und Wasser, trocknet über Magnesiumsulfat und dampft im Vakuum ein. Den Rückstand chromatographiert man mit Hexan/Essigester (9+1) an Kieselgel. Dabei erhält man 330 mg der Titelverbindung als farbloses Öl.

IR: 2945, 2860, 1732, 1599, 1688, 1495, 970/cm.

### Beispiel 2

(2E,13E)-(9R,11R,15R)-11,15-Dihydroxy-9-fluor-16-phenoxy-17,18,19,20-tetranor-2,13-prostadiensäure

102 mg des nach Beispiel 1 hergestellten Methylesters rührt man 5 Stunden mit 6 ml einer Lösung aus Kaliumhydroxid in Äthanol under Wasser (Herstellung: Man löst 2 g Kaliumhydroxid in 75 ml Äthanol und 25 ml Wasser). Anschließend säuert man mit 10%iger Zitronensäurelösung auf pH4 an, extrahiert dreimal mit Methylenchlorid, wäscht den organischen Extrakt einmal mit Sole, trocknet über Magnesiumsulfat und dampft im Vakuum ein. Nach Chromatographie des Rückstandes an Kieselgel mit Methylenchlorid/Isopropanol (92+8) als Elutionsmittel erhält man 84 mg der Titelverbindung als farbloses Öl.

IR: 3600, 3400 (breit), 2930, 2859, 1696, 1652, 1599, 1588, 1494, 970/cm.

### Beispiel 3

(2E,13E)-(9S,11R,15R)-11,15-Dihydroxy-9-Fluor-16-phenoxy-17,18,19,20-tetranor-2,13-prostadiensäure methylester

In Analogie zu Beispiel 1 erhält man aus 400 mg (13E)-(9S,11R,15R)-11,15-Bis-(tetrahydropyran-2-yloxy)-9-fluor-16-phenoxy-17,18,19,20-tetranor-13-prostensäuremethylester 180 mg (2E,13E)-(9S,11R,15R)-11,15-Bis-(tetrahydropyranyloxy)-9-fluor-16-phenoxy-17,18,19,20-tetranor-2,13-prostadiensäuremethylester.

Nach Abspaltung der Schutzgruppen gemäß Beispiel 1 erhält man 120 mg der Titelverbindung als farbloses Öl.

IR: 3600, 3410 (breit), 2950, 2860, 1711, 1657, 1600, 1589, 970/cm.

Das Ausgangsmaterial für die obige Titelverbindung wird wie folgt hergestellt:

3 a)

(13E)-(9S,11R,15R)-11,15-Bis-(tetrahydropyran-2-yloxy)-9-fluor-16-phenoxy-17,18,19,20-tetranor-13-prostensäuremethylester

In Analogue zu Beispiel 1 a) erhält man aus 380 mg (13E)-(9S,11R,15R)-11,15-Dihydroxy-9-fluor-16-phenoxy-17,18,19,20-tetranor-13-prostensäuremethylester 450 mg der Titelverbindung als farbloses Öl.

IR: 2946, 2860, 1731, 1600, 1589, 971/cm.

Beispiel 4

(2E,13E)-(9S,11R,15R)-11,15-Dihydroxy-9-fluor-16-phenoxy-17,18,19,20-tetranor-2,13-prostadiensäure

In Analogie zu Beispiel 2 erhält man aus 140 mg des nach Beispiel 3 hergestellten Methylesters 120 mg der Titelverbindung als farbloses Öl.

IR: 3610, 3410 (breit), 2931, 2860, 1697, 1652, 1600, 1589, 971/cm.

Beispiel 5

(2E,13E)-(9R,11R,15R)-11,15-Dihydroxy-16,16-dimethyl-9-fluor-2,13-prostadiensäuremethylester

In Analogie zu Beispiel 1 erhält man aus 250 mg (13E)-(9R,11R,15R)-16,16-Dimethyl-11,15-bis-(tetrahydropyran-2-yloxy)-9-fluor-13-prostensäuremethylester 203 mg (2E,13E)-(9R,11R,15R)-16,16-Dimethyl-11,15-bis-(tetrahydropyran-2-yloxy)-9-fluor-2,13-prostadiensäuremethylester.

Nach Abspaltung der Schutzgruppen gemäß Beispiel 1 erhält man 135 mg der Titelverbindung als farbloses Öl.

IR: 3600, 3400, 2960, 2936, 2875, 1718, 1656, 973/cm.

Das Ausgangsmaterial für die obige Titelverbindung wird wie folgt hergestellt:

5 a)

(13E)-(9R,11R,15R)-16,16-Dimethyl-11,15-bis-(tetrahydropyran-2-yloxy)-9-fluor-13-prostensäuremethylester

In Analogie zu Beispiel 1 a) erhält man aus 300 mg (13E)-(9R,11R,15R)-11,15-Dihydroxy-16,16-dimethyl-9-fluor-13-prostensäuremethylester 360 mg Titelverbindung als farbloses Öl.

IR: 2942, 2858, 1736, 973/cm.

Beispiel 6

(2E,13E)-(9R,11R,15R)-11,15-Dihydroxy-16,16-dimethyl-9-fluor-2,13-prostadiensäure

In Analogie zu Beispiel 2 erhält man aus 100 mg des nach Beispiel 5 hergestellten Methylesters 81 mg der Titelverbindung als farbloses Öl.

IR: 3605, 3410, 2960, 2932, 2876, 1699, 1650, 972/cm.

Beispiel 7

(2E,13E)-(9R,11R,15S,16RS)-11,15-Dihydroxy-9-fluor-16-methyl-2,13-prostadiensäuremethylester

In Analogie zu Beispiel 1 erhält man aus 300 mg (13E)-(9R,11R,15S,16RS)-11,15-Bis-(tetrahydropyran-2-yloxy)-9-fluor-16-methyl-13-prostensäuremethylester 235 mg (2E,13E)-(9R,11R,15,16RS)-11,15-Bis-(tetrahydropyran-2-yloxy)-9-fluor-16-methyl-2,13-prostadiensäuremethylester.

Nach Abspaltung der Schutzgruppen gemäß Beispiel 1 erhält man 145 mg der Titelverbindung als farbloses Öl.

IR: 3600, 3410, 2960, 2935, 2872, 1715, 1655, 972/cm.

Das Ausgangsmaterial für die obige Titelverbindung wird wie folgt hergestellt:

7 a)

(13E)-(9R,11R,15S,16RS)-11,15-Bis-(tetrahydropyran-2-yloxy)-9-fluor-16-methyl-13-prostensäuremethylester

In Analogie zu Beispiel 1 a) erhält man aus 310 mg (13E)-(9R,11R,15S,16RS)-11,15-Dihydroxy-9-fluor-16-methyl-13-prostensäuremethylester 359 mg der Titelverbindung als farbloses Öl.

IR: 2960, 2935, 2872, 1738, 976/cm.

Beispiel 8

(2E,13E)-(9R,11R,15S,16RS)-11,15-Dihydroxy-9-fluor-16-methyl-2,13-prostadiensäure

In Analogie zu Beispiel 2 erhält man aus 100 mg des nach Beispiel 7 hergestellten Methylesters 80 mg der Titelverbindung als farbloses Öl.

IR: 3605, 3400, 2960, 2935, 2875, 1700, 1652, 973/cm.

Beispiel 9

(2E,13E)-(9R,11R,15S,16RS)-11,15-Dihydroxy-16-19-dimethyl-9-fluor-2,13,18-prostatriensäuremethylester

In Analogie zu Beispiel 1 erhält man aus 300 mg (13E)-(9R,11R,15S,16RS)-11,15-Bis-(tetrahydropyran-2-yloxy)-16,19-dimethyl-9-fluor-13,18-prostadiensäuremethylester 230 mg (2E,13E)-(9R,11R,15S,16RS)-11,15-Bis-(tetrahydropyran-2-yloxy)-16,19-dimethhyl-9-fluor-2,13,18-prostatriensäuremethylester.

Nach Abspaltung der Schutzgruppen gemäß Beispiel 1 erhält man 132 mg der Titelverbindung als farbloses Öl.

IR: 3600, 3410, 2960, 2932, 2878, 1715, 1655, 974/cm.

Das Ausgangsmaterial für die obige Titelverbindung wird wie folgt hergestellt:

9 a)

(13E)-(9R,11R,15S,16RS)-11,15-Bis-(tetrahydropyran-2-yloxy)-16,19-dimethyl-9-fluor-13,18-prostadien säuremethylester

In Analogie zu Beispiel 1 a) erhält man aus 250 mg (13E)-(9R,11R,15S,16RS)-11,15-Dihydroxy-16,19-dimethyl-9-fluor-13,18-prostadiensäuremethylester 355 mg der Titelverbindung als farbloses Öl.

IR: 2962, 2855, 1736, 974/cm.

Beispiel 10

(2E,13E)-(9R,11R,15S,16RS)-11,15-Dihydroxy-16,19-dimethyl-9-fluor-2,13,18-prostatriensäure

In Analogie zu Beispiel 2 erhält man aus 130 mg des nach Beispiel 9 hergestellten Methylesters 105 mg der Titelverbindung als farbloses Öl.

IR: 3605, 3405, 2959, 2935, 2958, 1700, 1649, 974/cm.

Beispiel 11

(2E,13E)-(9R,11R,15RS)-11,15-Dihydroxy-9-fluor-15-methyl-2,13-prostadiensäuremethylester

In Analogie zu Beispiel 1 erhält man aus 200 mg (13E)-(9R,11R,15RS)-11,15-Bis-(tetrahydropyran-2-yloxy)-9-fluor-15-methyl-13-prostensäuremethylester 150 mg (2E,13E)-(9R,11R,15RS)-11,15-Bis-(Tetrahydropyran-2-yloxy)-9-fluor-15-methyl-2,13-prostadiensäuremethylester.

Nach Abspaltung der Schutzgruppen gemäß Beispiel 1 erhält man 80 mg der Titelverbindung als farbloses Öl.

IR: 3600, 3410 (breit), 2960, 2881, 1715, 1658, 974/cm.

Das Ausgangsmaterial für die obige Titelverbindung wird wie folgt hergestellt:

11 a)

(13E)-(9R,11R,15RS)-11,15-Bis-(tetrahydropyran-2-yloxy)-9-fluor-15-methyl-13-prostensäuremethylester

Eine Lösung von 400 mg (5Z,13E)-(9R,11R,15RS)-11,15-Bis-(tetrahydropyran-2-yloxy)-9-fluor-15-methyl-5,13-prostadiensäuremethylester in 50 ml Ethylacetat wird mit 40 mg Palladium/10%ig auf Kohle unter einen Wasserstoffatmosphäre bei 0°C geschüttelt Nach der Aufnahme von 1 Äquivalent Wasserstoff wird vom Katalysator abfiltriert und im Vakuum eingedampft. Der Rückstand wird an Kieselgel absorbiert. Mit Hexan/5—20% Diethylether eluiert man 300 mg der Titelverbindung als farbloses Öl.

IR: 2958, 2850, 1738, 976/cm.

Beispiel 12

(2E,13E)-(9R,11R,15RS)-11,15-Dihydroxy-9-fluor-15-methyl-2,13-prostadiensäure

In Analogie zu Beispiel 2 erhält man aus 70 mg des nach Beispiel 11 hergestellten Methylester 55 mg der Titelverbindung als farbloses Öl.

IR: 3600, 3410 (breit), 2960, 2855, 1701, 1652, 974/cm.

Beispiel 13

(2E,13E)-(9R,11R,15S)-11,15-Dihydroxy-9-fluor-2,13-prostadiensäuremethylester

In Analogie zu Beispiel 1 erhält man aus 300 mg (13E)-(9R,11R,15S)-11,15-Bis-(tetrahydropyran-2-yloxy)-9-fluor-13-prostensäuremethylester 235 mg (2E,13E)-(9R,11R,15S)-11,15-Bis-(tetrahydropyran-2-yloxy)-9-fluor-2,13-prostadiensäuremethylester.

Nach Abspaltung der Schutzgruppen gemäß Beispiel 1 erhält man 140 mg der Titelverbindung als farbloses Öl.

IR: 3600, 3410, 2958, 2862, 1718, 1656, 974/cm.

Das Ausgangsmaterial für die obige Titelverbindung wird wie folgt hergestellt:

13 a)

(13E)-(9R,11R,15S)-11,15-Bis-(tetrahydropyran-2-yloxy)-9-fluor-13-prostensäuremethylester

In Analogie zu Beispiel 11 a hydriert man 500 mg (5Z,13E)-(9R,11R,15S)-11,15-Bis-(tetrahydropyran-2-yloxy)-9-fluor-5,13-prostadiensäuremethylester und erhält 405 mg der Titelverbindung als farbloses Öl.

IR: 2960, 1738, 976/cm.

Beispiel 14

(2E,13E)-(9R,11R,15S)-11,15-Dihydroxy-9-fluor-2,13-prostadiensäure

In Analogie zu Beispiel 2 erhält man aus 100 mg des nach Beispiel 13 hergestellten Methylesters 86 mg der Titelverbindung als farbloses Öl.

IR: 3600, 3410 (breit), 2957, 1698, 1651, 976/cm.

Beispiel 15

(2E,13E)-(9R,11R,15R)-9-Chlor-11,15-dihydroxy-16-phenoxy-17,18,19,20-tetranor-2,13-prostadiensäuremethylester

Man löst 0,36 ml Diisopropylamin in 3 ml abs. Tetrahydrofuran und versetzt bei −20°C mit 1,04 ml einer 1,6 molaren Butyllithiumlösung in Hexan und rührt 30 Minuten bei −20°C unter Argon. In diese Lösung tropft man bei −70°C eine Lösung von 500 mg (13E)-(9R,11R,15R)-11,15-Bis-(tetrahydropyran-2-yloxy)-9-chlor-16-phenoxy-17,18,19,20-tetranor-13-prostensäuremethylester in 5 ml abs. Tetrahydrofuran, rührt 30 Minuten bei −70°C und tropft dann eine Lösung von 434 mg Diphenyldiselenid in 3 ml abs. Tetrahydrofuran zu. Nach einer Stunde bei −40°C wird mit Wasser verdünnt, mit 3%iger Schwefelsäure auf pH6 gestellt, einige Male mit Äther extrahiert, der Extrakt mit Sole geschüttelt, über Magnesiumsulfat getrocknet und im Vakuum eingedampft.

Das Rohprodukt (371 mg gelbes Öl) löst man in 8 ml Dichlormethan und 0,2 ml Pyridin, versetzt mit 0,2 ml 30%iger Wasserstoffperoxidlösung und rührt 1,5 Stunden bei Raumtemperatur. Dann wird mit Dichlormethan verdünnt, mit Natriumhydrogencarbonatlösung und Sole geschüttelt, über Magnesiumsulfat getrocknet und im Vakuum eingedampft. Zur Reinigung adsorbiert man an Kieselgel und eluiert mit Hexan-Ethylacetat (85:15) und erhält 205 mg (2E,13E)-(9R,11R,15R)-11,15-Bis-(tetrahydropyran-2-yloxy)-9-chlor-16-phenoxy-17,18,19,20-tetranor-2,13-prostadiensäuremethylester als Öl.

IR: 2958, 2849, 1718, 1658, 1600, 1580, 874/cm.

Zur Abspaltung der Schutzgruppen rührt man 205 mg der vorstehend hergestellten $\Delta^2$-Verbindung über Nacht bei Raumtemperatur mit 10 ml Essigsäure/Wasser/Tetrahydrofuran (65/35/15) und dampft anschließend im Vakuum ein. Man reinigt an Kieselgel mit Dichlormethan/Aceton (8:2) und erhält 128 mg der Titelverbindung als farbloses Öl.

IR: 3600, 3410 (breit), 2955, 2860, 1710, 1655, 1600, 1585, 972/cm.

Das Ausgangsmaterial für die obige Titelverbindung wird wie folgt hergestellt:

15 a)

(13E)-(9R,11R,15R)-11,15-Bis-(tetrahydropyran-2-yloxy)-9-chlor-16-phenoxy-17,18,19,20-tetranor-13-prostensäuremethylester

Zu einer Lösung von 450 mg (13E)-(9R,11R,15R)-9-Chlor-11,15-dihydroxy-16-phenoxy-17,18,19,20-tetranor-13-prostensäuremethylester in 20 ml Dichlormethan gibt man bei 0°C 290 mg Dihydropyran und 5 mg p-Toluolsulfonsäure. Nach 30 Minuten wird mit Dichlormethan verdünnt, mit Natriumhydrogencarbonatlösung und Sole geschüttelt, über Magnesiumsulfat, getrocknet und im Vakuum eingedampft. Den Rückstand chromatographiert man mit Hexan/Ethylacetat (9:1) an Kieselgel und erhält 570 mg der Titelverbindung als Farbloses Öl.

IR: 2952, 2862, 1738, 1600, 1585, 972/cm.

Beispiel 16

(2E,13E)-(9R,11R,15R)-9-Chlor-11,15-dihydroxy-16-phenoxy-17,18,19,20-tetranor-2,13-prostadiensäure

In Analogie zu Beispiel 2 erhält man aus 90 mg des nach Beispiel 15 hergestellten Methylesters 75 mg der Titelverbindung als farbloses Öl.

IR: 3600, 3405 (breit), 2958, 2932, 2852, 1698, 1650, 1600, 1588, 972/cm.

Beispiel 17

(2E,13E)-(9S,11R,15R)-9-Chlor-11,15-dihydroxy-16-phenoxy-17,18,19,20-tetranor-2,13-prostadiensäuremethylester

In Analogie zu Beispiel 15 erhält man aus 350 mg (13E)-(9S,11R,15R)-11,15-Bis-(tetrahydropyran-2-yloxy)-9-chlor-16-phenoxy-17,18,19,20-tetranor-13-prostensäuremethylester 280 mg (2E,13E)-(9S,11R,15R)-11,15-Bis-(tetrahydropyran-2-yloxy)-9-chlor-16-phenoxy-17,18,19,20-tetranor-2,13-prostadiensäuremethylester.

Nach Abspaltung der Schutzgruppen gemäß Beispiel 15 erhalt man 160 mg der Titelverbindung als farbloses Öl.

IR: 3600, 3405 (breit), 2958, 2932, 2860, 1712, 1658, 1600, 1588, 976/cm.

Das Ausgangsmaterial wird wie folgt hergestellt:

17 a)

(13E)-(9S,11R,15R)-11,15-Bis-(tetrahydropyran-2-yloxy)-9-chlor-16-phenoxy-17,18,19,20-tetranor-13-prostensäuremethylester

In Analogie zu Beispiel 15 a) erhält man aus 350 mg (13E)-(9S,11R,15R)-9-Chlor-11,15-dihydroxy-16-phenoxy-17,18,19,20-tetranor-13-prostensäuremethylester 430 mg der Titelverbindung als farbloses Öl.

IR: 2955, 2860, 1738, 1600, 1588, 974/cm.

Beispiel 18

(2E,13E)-(9S,11R,15R)-9-Chlor-11,15-dihydroxy-16-phenoxy-17,18,19,20-tetranor-2,13-prostadiensäure

In Analogie zu Beispiel 2 erhält man aus 150 mg des nach Beispiel 17 hergestellten Methylesters 130 mg der Titelverbindung als farbloses Öl.

IR: 3605, 3410 (breit), 2952, 2930, 2862, 1700, 1651, 1600, 1585, 874/cm.

Beispiel 19

(2E,13E)-(9R,11R,15R)-9-Chlor-11,15-Dihydroxy-16,16-dimethyl-2,13-prostadiensäuremethylester

In Analogie zu Beispiel 15 erhält man aus 250 mg (13E)-(9R,11R,15R)-11,15-Bis-(tetrahydropyran-2-yloxy)-9-chlor-16,16-dimethyl-13-prostensäuremethylester 180 mg (2E,13E)-(9R,11R,15R)-11,15-Bis-(tetrahydropyran-2-yloxy)-9-chlor-16,16-dimethyl-2,13-prostadiensäuremethylester.

Nach Abspaltung der Schutzgruppen gemäß Beispiel 15 erhält man 85 mg der Titelverbindung als farbloses Öl.

IR: 3600, 3405 (breit), 2960, 2935, 2872, 1715, 1658, 976/cm.

Das Ausgangsmaterial wird wie folgt hergestellt:

19 a)

(13E)-(9R,11R,15R)-11,15-Bis-(tetrahydropyran-2-yloxy)-9-chlor-16,16-dimethyl-13-prostensäuremethylester

In Analogie zu Beispiel 15 a) erhält man aus 220 mg (13E)-(9R,11R,15R)-9-Chlor-11,15-dihydroxy-16,16-dimethyl-13-prostensäuremethylester 280 mg der Titelverbindung als farbloses Öl.

IR: 2955, 2870, 1738, 974/cm.

Beispiel 20

(2E,13E)-(9R,11R,15R)-9-Chlor-11,15-dihydroxy-16,16-dimethyl-2,13-prostadiensäure

In Analogie zu Beispiel 2 erhält man aus 80 mg des nach Beispiel 19 hergestellten Methylesters 65 mg der Titelverbindung als farbloses Öl.

IR: 3605, 3420 (breit), 2960, 2872, 1701, 1652, 974/cm.

Beispiel 21

(2E,13E)-(9R,11R,15S,16RS)-9-Chlor-11,15-dihydroxy-16-methyl-2,13-prostadiensäuremethylester

In Analogie zu Beispiel 15 erhält man aus 200 mg (13E)-(9R,11R,15S,16RS)-11,15-Bis-(tetrahydropyran-2-yloxy)-9-chlor-16-methyl-13-prostensäuremethylester 160 mg (2E,13E)-(9R,11R,15S,16RS)-11,15-Bis-(tetrahydropyran-2-yloxy)-9-chlor-16-methyl-2,13-prostadiensäuremethylester.

Nach Abspatlung der Schutzgruppen gemäß Beispiel 15 erhält man 80 mg der Titelverbindung als farbloses Öl.

IR: 3600, 3405 (breit), 2952, 2934, 2870, 1716, 1658, 974/cm.

Das Ausgangsmaterial wird wie folgt hergestellt:

21 a)

(13E)-(9R,11R,15S,16RS)-11,15-Bis-(tetrahydropyran-2-yloxy)-9-chlor-16-methyl-13-prostensäuremethylester

In Analogie zu Beispiel 15 a) erhält man aus 200 mg (13E)-(9R,11R,15S,16RS)-9-Chlor-11,15-dihydroxy-16-methyl-13-prostensäuremethylester 255 mg der Titelverbindung als farbloses Öl.

IR: 2958, 2862, 1735, 976/cm.

Beispiel 22

(2E,13E)-(9R,11R,15S,16RS)-9-Chlor-11,15-dihydroxy-16-methyl-2,13-prostadiensäure

In Analogue zu Beispiel 2 erhält man aus 70 mg des nach Beispiel 21 hergestellten Methylesters 50 mg der Titelverbindung als farbloses Öl.

IR: 3600, 3410 (breit), 2958, 2935, 2872, 1698, 1651, 974/cm.

Beispiel 23

(2E,13E)-(9R,11R,15RS)-9-Chlor-11,15-dihydroxy-15-methyl-2,13-prostadiensäuremethylester

In Analogie zu Beispiel 15 erhält man aus 400 mg (13E)-(9R,11R,15RS)-11,15-Bis-tetrahydropyran-2-yloxy)-9-chlor-15-methyl-13-prostensäuremethylester 320 mg (2E,13E)-(9R,11R,15RS)-11,15-Bis-(tetrahydropyran-2-yloxy)-9-chlor-15-methyl)-2,13-prostadiensäuremethylester.

Nach Abspaltung der Schutzgruppen gemäß Beispiel 15 erhalt man 180 mg der Titelverbindung als farbloses Öl.

IR: 3600, 3400, 2955, 2870, 1718, 1657, 976/cm.

Das Ausgangsmaterial wird wie folgt hergestellt:

23 a)

(13E)-(9R,11R,15RS)-11,15-Bis-(tetrahydropyran-2-yloxy)-9-chlor-15-methyl-13-prostensäuremethylester

In Analogie zu Beispiel 11 a) hydriert man selektiv 600 mg (5Z, 13E)-(9R,11R,15RS)-11,15-Bis-(tetrahydropyran-2-yloxy)-9-chlor-15-methyl-5,13-prostadiensäuremethylester und erhält 480 mg der Titelverbindung als farbloses Öl.

IR: 2960, 2872, 1735, 974/cm.

Beispiel 24

(2E,13E)-(9R,11R,15RS)-9-chlor-11,15-dihydroxy-15-methyl-2,13-prostadiensäure

In Analogie zu Beispiel 2 erhält man aus 150 mg des nach Beispiel 23 hergestellten Methylesters 125 mg der Titelverbindung als farbloses Öl.

IR: 3605, 3410 (breit), 2958, 2930, 2875, 1700, 1652, 976/cm.

Beispiel 25

(2E,13E)-(9R,11R,15S)-9-chlor-11,15-dihydroxy-2,13-prostadiensäuremethylester

In Analogie zu Beispiel 15 erhält man aus 250 mg (13E)-(9R,11R,15S)-11,15-Bis-(tetrahydropyran-2-yloxy)-9-chlor-13-prostensäuremethylester 205 mg (2E,13E)-(9R,11R,15S)-11,15-Bis-(tetrahydropyran-2-yloxy)-9-chlor-2,13-prostadiensäuremethylester.

Nach Abspaltung der Schutzgruppen gemäß Beispiel 15 erhält man 125 mg der Titelverbindung als farbloses Öl.

IR: 3605, 3410 (breit), 2959, 2938, 2870, 1715, 1655, 974/cm.

Das Ausgangsmaterial wird wie folgt hergestellt:

25 a)

(13E)-(9R,11R,15S)-11,15-Bis-(tetraphydropyran-2-yloxy)-9-chlor-13-prostensäuremethylester

In Analogie zu Beispiel 11 a) hydriert man selektiv 415 mg (5Z,13E)-(9R,11R,15S)-11,15-Bis-(tetrahydropyran-2-yloxy)-9-chlor-5,13-prostadiensäuremethylester und erhält 337 mg der Titelverbindung als farbloses Öl.

IR: 2953, 2868, 1738, 976/cm.

Beispiel 26

(2E,13E)-(9R,11R,15S)-9-chlor-11,15-dihydroxy-2,13-prostadiensäure

In Analogie zu Beispiel 2 erhält man aus 97 mg des nach Beispiel 25 hergestellten Methylesters 78 mg der Titelverbindung als farbloses Öl.

IR: 3605, 3410 (breit), 2958, 2935, 2873, 1700, 1651, 975/cm.

Beispiel 27

(2E,5Z,13E)-(9R,11R,15R)-9-Chlor-11,15-dihydroxy-16,16-dimethyl-2,5,13-prostatriensäuremethylester

In Analogie zu Beispiel 15 erhält man aus 285 mg (5Z,13E)-(9R,11R,15R)-11,15-Bis-(tetrahydropyran-2-yloxy)-9-chlor-16,16-dimethyl-5,13-prostadiensäuremethylester 222 mg (2E,5Z,13E)-(9R,11R,15R)11,15-Bis-(tetrahydropyran-2-yloxy)-9-chlor-16,16-dimethyl-2,5,13-prostatriensäuremethylester.

Nach Abspaltung der Schutzgruppen gemäß Beispiel 15 erhält man 125 mg der Titelverbindung als farbloses Öl.

IR: 3600, 3405, 2958, 2932, 2873, 1715, 1657, 975/cm.

Das Ausgangsmaterial wird wie folgt hergestellt:

27 a)

(5Z,13E)-(9R,11R,15R)-11,15-Bis-(tetrahydropyran-2-yloxy)-9-chlor-16,16-dimethyl-5,13-prostadiensäuremethylester

In Analogie zu Beispiel 15 a) erhält man aus 240 mg (5Z,13E)-(9R,11R,15R)-9-Chlor-11,15-dihydroxy-16,16-dimethyl-5,13-prostadiensäuremethylester 328 mg der Titelverbindung als farbloses Öl.

IR: 2957, 2862, 1738, 974/cm.

Beispiel 28

(2E,5Z,13E)-(9R,11R,15R)-9-Chlor-11,15-dihydroxy-16,16-dimethyl-2,5,13-prostatriensäure

Man löst 1,10 g Lipase (EC 3.1.1.3, 20 Einheiten/mg) in 200 ml Wasser und leitet ständig einen langsamen Argonstrom durch die Lösung. Durch Zugabe von 0.01 N Natronlauge wird ein pH von 7,0 eingestellt. Danach wird eine Lösung von 125 mg des nach Beispiel 27 hergestellten Methylesters in 0,5 ml Ethanol zugetropft und 24 Stunden bei Raumtemperatur gerührt, wobei der pH-Wert der Lösung durch Zugabe von 0.01 N Natronlauge bei 7,2 gehalten wird. Zur Isolierung wird mit Citronensäure auf pH4 eingestellt und mehrmals mit Ethylacetat extrahiert. Die vereinigten Extrakte werden mit Sole geschüttelt, über Magnesiumsulfat getrocknet und im Vakuum eingedampft. Der Rückstand wird durch Chromatographie an Kieselgel mit Hexan/Ethylacetat gereinigt. Man erhält als unpolarere Fraktion 25 mg nicht umgesetztes Startmaterial und als polarere Fraktion 76 mg der Titelverbindung als farbloses Öl.

IR: 3605, 3405 (breit), 2957, 2932, 2875, 1701, 1651, 976/cm.

Beispiel 29

(2E,5Z,13E)-(9R,11R,15R)-11,15-Dihydroxy-16,16-dimethyl-9-fluor-2,5,13-prostatriensäuremethylester

In Analogie zu Beispiel 15 erhält man aus 275 mg (5Z,13E)-(9R,11R,15R)-11,15-Bis-(tetrahydropyran-2-yloxy)-16,16-dimethyl-9-fluor-5,13-prostadiensäuremethylester 219 mg (2E,5Z,13E)-(9R,11R,15R)-11,15-Bis-(tetrahydropyran-2-yloxy)-16,16-dimethyl-9-fluor-2,5,13-prostratriensäuremethylester.

Nach Abspaltung der Schutzgruppen gemäß Beispiel 15 erhält man 130 mg der Titelverbindung als farbloses Öl.

IR: 3605, 3400 (breit), 2961, 2935, 2875, 1716, 1658, 976/cm.
Das Ausgangsmaterial wird wie folgt hergestellt:

29 a)
(5Z,13E)-(9R,11R,15R)-11,15-Bis-(tetrahydropyran-2-yloxy)-16,16-dimethyl-9-fluor-5,13-prostadiensäure-methylester

In Analogie zu Beispiel 15 a) erhält man aus 210 mg (5Z,13E)-(9R,11R,15R)-11,15-Dihydroxy-16,16-dimethyl-9-fluor-5,13-prostadiensäuremethylester 280 mg der Titelverbindung als farbloses Öl.
IR: 2957, 2860, 1738, 976/cm.

Beispiel 30

(2E,5Z,13E)-(9R,11R,15R)-11,15-Dihydroxy-16,16-dimethyl-9-fluor-2,5-13-prostatriensäure

In Analogie zu Beispiel 28 erhält man aus 100 mg des nach Beispiel 29 hergestellten Methylesters durch Umsetzung mit Lipase 60 mg der Titelverbindung als farbloses Öl.
IR: 3605, 3410 (breit), 2955, 2932, 2872, 1698, 1650, 975/cm.

Beispiel 31

(2E,13E)-(9R,11R,15R)-11,15-Dihydroxy-9-fluor-16-phenoxy-17,18-19,20-tetranor-2,13-prostadiensäure-methylsulfonamid

Zu einer Lösung von 194 mg (2E,13E)-(9R,11R,15R)-11,15-Dihydroxy-9-fluor-16-phenoxy-17,18,19,20-tetranor-2-13-prostadiensäure in 3 ml Dimethylformamid gibt man 53 mg Triäthylamin und 75 mg Chlorameisensäureisobutylester und rührt 30 Minuten bei 0°C. Dann versetzt man mit 236 mg Methylsulfonamid-natriumsalz und 0,5 ml Hexanmethylphosphorsäuretriamid und rührt über Nacht bei 25°C. Zum Aufarbeiten verdünnt man mit Wasser, säuert mit verdünnter Schwefelsäure auf pH 3 an und extrahiert mehrmals mit Ethylacetat. Die vereinigten Extrakte werden mit Sole gewaschen, über Magnesiumsulfat getrocknet und im Vakuum eingedampft. Den Rückstand chromatographiert man an Kieselgel mit Hexan/Ethylacetat und erhält 135 mg der Titelverbindung als farbloses Öl.
IR: 3600, 3410, 2956, 2850, 1701, 1658, 1600, 1589, 976/cm.

Beispiel 32

(2E,13E)-(9R,11R,15R)-9-Chlor-11,15-dihydroxy-16,16-dimethyl-2,13-prostadiensäure-N-acetylamid

250 mg (2E,13E)-(9R,11R,15R)-11,15-Bis-(tetrahydropyran-2-yloxy)-9-chlor-16,16-dimethyl-2,13-prostadiensäuremethylester versetzt man mit einer Lösung von 60 mg Kaliumhydroxid in 2,5 ml Ethanol und 0,75 ml Wasser und rührt 2 Stunden bei Raumtemperatur. Anschließend engt man im Vakuum ein, verdünnt mit 100 ml Citratpuffer (pH 4), extrahiert mehrmals mit Dichlormethan, trocknet über Magnesiumsulfat und dampft im Vakuum ein. Zum Trocknen wird der Rückstand jeweils dreimal in 1 ml Benzol gelöst und die Lösung im Vakuum eingedampft. Die so erhaltene Prostaglandinsäure löst man in 2 ml Acetonitril und versetzt nacheinander bei 0°C mit 125 mg Triathylamin und 200 mg Acetylisocyanat. Man läßt 2 Stunden bei 20°C stehen, dampft im Vakuum ein, versetzt mit Wasser, säuert mit Citronensäure auf pH 5 an und extrahiert mit Äther. Den Extrakt wäscht man mit Sole, trocknet über Magnesiumsulfat und dampft im Vakuum ein. Zur Abspaltung der Schutzgruppen rührt man den Rückstand 4 Stunden bei 40°C mit 5 ml einer Mischung aus Essigsäure-Wasser-Tetrahydrofuran (65/35/10), dampft im Vakuum ein und absorbiert den Rückstand an 50 g Kieselgel. Durch Elution mit Hexan/Ethylacetat erhält man 115 mg der Titelverbindung als hellgelbes Öl.
IR: 3600, 3410, 2956, 2868, 1702, 1658, 1505, 978/cm.

Beispiel 33

(2E,13E)-(9R,11R,15R)-11,15-Dihydroxy-9-fluor-16-phenoxy-17,18,19,20-tetranor-2,13-prostadiensäure-tris(hydroxymethyl)aminomethansalz

Zu einer Lösung von 233 mg (2E,13E)-(9R,11R,15R)-11,15-Dihydroxy-9-fluor-16-phenoxy-17,18,19,20-tetranor-2,13-prostadiensäure in 35 ml Acetonitril fügt man unter Rühren eine Lösung von 75 mg Tris(hydroxymethyl)aminomethan in 0,25 ml Wasser und läßt innerhalb von 5 Stunden unter Rühren auf Raumtemperatur abkühlen. Man saugt das ausgefallene Pulver ab und erhält nach Trocknen im Vakuum 222 mg der Titelverbindung.

## Patentansprüche

1. 9-Halogen-$\Delta^2$-prostanderivate der allgemeinen Formel I

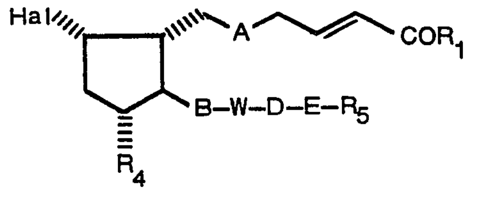

(I),

worin

Hal ein α- oder β-ständiges Fluor- oder Chloratom sein kann,

$R_1$ den Rest $OR_2$ mit $R_2$ in der Bedeutung eines Wasserstoffatoms, eines $C_{1-10}$-Alkyls, $C_{3-10}$-Cycloalkyls, Phenyls oder eines 5- oder 6-gliedrigen heterocyclischen Restes oder $R_1$ den Rest $NHR_3$ mit $R_3$ in der Bedeutung eines organischen Carbonsäure- oder Sulfonsäurerestes mit 1—15 C-Atomen oder des Restes $R_2$ und

A eine $—CH_2—CH_2—$ oder cis-CH=CH-Gruppe,

B eine $—CH_2—CH_2—$, oder trans-CH=CH— oder eine —C≡C-Gruppe,

W eine

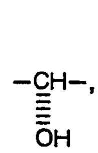 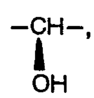 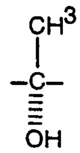 or 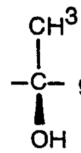 group,

in denen die OH-Gruppen durch Tetrahydropyranyloxy, Tetrahydrofuranyloxy, α-Ethoxyethoxy, Trimethylsilyloxy, Dimethyl-tert.-butylsilyloxy, Acetoxy, Propionyloxy, Butyryloxy oder Benzoyloxy ersetzt werden können,

D und E gemeinsam eine direkte Bindung oder

D eine gerad- oder verzweigtkettige gesättigte Alkylengruppe mit 1—5 C-Atomen, die gegebenenfalls durch Fluoratome substituiert sein kann,

E ein Sauerstoff- oder Schwefelatom, eine direkte Bindung, eine —C≡C-Bindung oder eine $—CR_6{\equiv}CR_7$-Gruppe darstellt, wobei $R_6$ ein Wasserstoffatom oder eine Alkylgruppe mit 1—6 mit C-Atomen und $R_7$ ein Wasserstoffatom, eine Alkylgruppe mit 1—6 C-Atomen oder ein Halogenatom bedeuten,

$R_4$ eine freie Hydroxygruppe, die durch Tetrahydropyranyloxy, Tetrahydrofuranyloxy, α-Ethoxyethoxy, Trimethylsilyloxy, Dimethyl-tert.-butylsilyloxy, Acetoxy, Propionyloxy, Butyryloxy oder Benzoyloxy ersetzt werden kann.

$R_5$ ein Wasserstoffatom, eine $C_{1-6}$-Alkyl-, eine Halogen-substituierte $C_{1-6}$-Alkyl, eine $C_{1-6}$-cyclopalkyl-, eine Phenyl- oder eine 5- oder 6-gliedrige heterocyclische Gruppe und, falls $R_2$ die Bedeutung eines Wasserstoffatoms hat, deren Salze mit physiologisch verträglichen Basen bedeuten.

2. Verfahren zur Herstellung von 9-Halogen-$\Delta^2$-prostanderivaten der allgemeinen Formel I, dadurch gekennzeichnet, daß man in an sich bekannter Weise in eine Verbindung der allgemeinen Formel II,

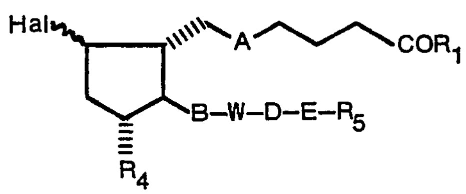

(II),

eine Doppelbindung in 2-Stellung einführt, indem man mit einem Lithiumdialkylamid der allgemeinen Formel III,

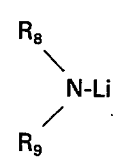

(III),

worin $R_8$ und $R_9$ eine Alkylgruppe mit 1—10 Kohlenstoffatomen oder eine Cycloalkylgruppe mit 3—6 Kohlenstoffatomen oder Trialkylsilylgruppe mit Alkyl in der Bedeutung $C_1$—$C_4$-Alkyl darstellen, umsetzt, anschließend mit einer Phenylverbindung der allgemeinen Formel IV,

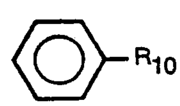

(IV),

worin $R_{10}$ die Reste

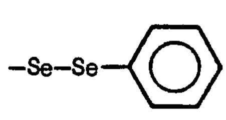 , 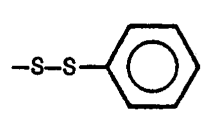 oder SeBr

oder SeBr darstellt, behandelt und anschließend in beliebiger Reihenfolge eine oxidative Eliminierung durchführt und/oder gegebenenfalls Isomere trennt und/oder geschützte Hydroxygruppen freisetzt und/oder freie Hydroxygruppen verestert, veräthert und/oder eine freie Carboxylgruppe verestert und/oder eine veresterte Carboxylgruppe verseift oder eine Carboxylgruppe in ein Amid oder mit einer physiologisch verträglichen Base in ein Salz überführt.

3. Arzneimittel, bestehend aus einer oder mehreren Verbindungen der Formel I und üblichen Hilfs- und Trägerstoffen.

**Revendications**

1. Dérivés d'halogéno-9Δ²-prostanes qui répondent à la formule générale I:

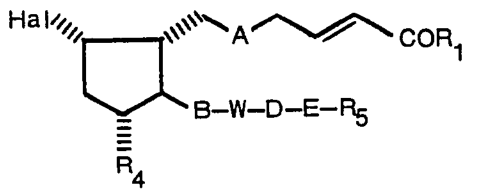

(I),

dans laquelle:

Hal représente un atome de fluor ou de chlore ayant la configuration α ou la configuration β,

$R_1$ représente un radical $—OR_2$ dans lequel $R_2$ représente un atome d'hydrogène, un alkyle en $C_1—C_{10}$, un cycloalkyle en $C_3—C_{10}$, un phényle ou un radical hétérocyclique à 5 ou 6 maillons, ou $R_1$ représente un radical $—NHR_3$ dans lequel $R_3$ désigne un radical d'acide organique carboxylique ou sulfonique contenant de 1 à 15 atomes de carbone ou a la signification de $R_2$,

A représente un radical $—CH_2—CH_2—$ ou un radical $—CH=CH—$ cis,

B représente un radical $—CH_2—CH_2—$, un radical $—CH=CH—$ trans ou un radical $—C≡C—$,

W représente l'un des radicaux

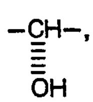 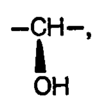 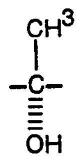 or 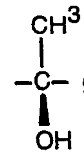 group,

dans lesquels chacun des radicaux —OH peut étre remplacé par un radical tétrahydropyrannyloxy, tétrahydrofurannyloxy, α-éthoxy-éthoxy, triméthylsilyloxy, diméthyl-tert-butylsilyloxy, acétoxy, propionyloxy, butyryloxy ou benzoyloxy,

D et E représentent ensemble une liaison directe ou

D représente un radical alkylène saturé linéaire ou ramifié, qui contient de 1 à 5 atomes de carbone et qui peut éventuellement porter des atomes de fluor,

E représente un atome d'oxygène ou de soufre, une liaison directe, une liaison $—C≡C—$ ou un radical $—CR_6=CR_7—$ dans lequel $R_6$ désigne un atome d'hydrogène ou un alkyle contenant de 1 à 6 atomes de carbone et $R_7$ un atome d'hydrogène, un alkyle contenant de 1 à 6 atomes de carbone ou un atome d'halogène,

$R_4$ représente un radical hydroxy libre, lequel peut être remplacé par un radical tétrahydropyrannyloxy, tétrahydrofurannyloxy, α-éthoxy-éthoxy, triméthylsilyloxy, diméthyl-tert-butyl-silyloxy, acétoxy, propionyloxy, butyryloxy ou benzoyloxy, et

$R_5$ représente un atome d'hydrogène, un alkyle en $C_1—C_6$, un alkyle en $C_1—C_6$ porteur d'un halogène, un cycloalkyle en $C_3—C_6$, un phényle ou un radical hétérocyclique à 5 ou 6 maillons, et, dans le cas où $R_2$ désigne un atome d'hydrogène, les sels que ces composés forment avec des bases acceptables du point de vue physiologique.

2. Procédé pour préparer des dérivés d'halogéno-9Δ²-prostanes de formule générale I, procédé caractérisé en ce que, en opérant de manière connue, on introduit une double liaison à la position 2 dans un composé de formule générale II:

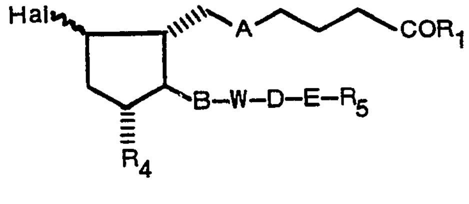

(II),

en le faisant réagir avec un dialkylamidure de lithium répondant à la formule générale III:

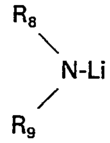

(III)

dans laquelle $R_8$ et $R_9$ représentent chacun un alkyle contenant de 1 à 10 atomes de carbone, un cycloalkyle contenant de 3 à 6 atomes de carbone ou un trialkylsilyle dont chacun des alkyles contient de 1 à 4 atomes de carbone, puis en traitant par un composé phénylique répondant à la formule générale IV;

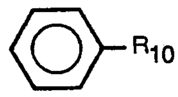

(IV),

dans laquelle $R_{10}$ représente un radical:

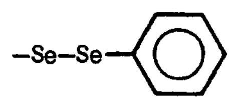 , 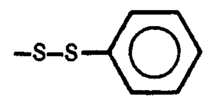 ou -SeBr,

après quoi, en adoptant l'ordre que l'on veut, on effectue une élimination par oxydation et/ou on sépare éventuellement des isomères et/ou on libère des radicaux hydroxy protégés et/ou on estérifie ou éthérifie des radicaux hydroxy libres et/ou on estérifie un radical carboxy libre et/ou on saponifie un radical carboxy estérifié ou on transforme un radical carboxy en un amide ou, au moyen d'une base acceptable du point de vue physiologique, en un sel.

3. Médicament constitué d'un ou de plusieurs composés de formule I et d'adjuvants et excipients usuels.

## Claims

1. 9-Halo-$\Delta^2$-prostane derivatives of general formula I

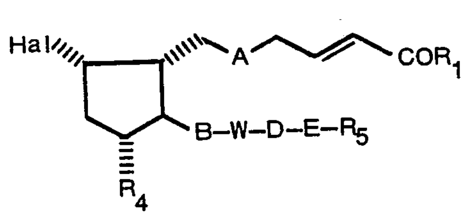

(I),

wherein

Hal may be a fluorine or chlorine atom in the α- or β-configuration,

$R_1$ is the radical $OR_2$, $R_2$ being a hydrogen atom, a $C_{1-10}$-alkyl, $C_{3-10}$-cycloalkyl, phenyl or a 5- or 6-membered heterocyclic radical, or $R_1$ is the radical $NHR_3$, $R_3$ being an organic carboxylic acid or sulphonic acid radical having from 1 to 15 carbon atoms or being as defined for the radical $R_2$ and

A is a $—CH_2—CH_2—$ or *cis*-CH=CH— group,

B is a $—CH_2—CH_2—$ or *trans*-CH=CH— or a —C≡C— group,

W is a

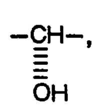, 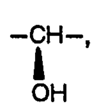, 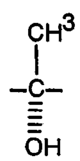 or 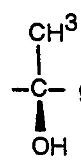 group,

wherein the hydroxy groups may be replaced by tetrahydropyranyloxy, tetrahydrofuranyloxy, α-ethoxyethoxy, trimethylsilyloxy, dimethyl-tert.-butylsilyloxy, acetoxy, propionyloxy, butyryloxy or benzoyloxy,

D and E together are a direct bond or D is a straight-chain or branched-chain saturated alkylene group having from 1 to 5 carbon atoms which may optionally be substituted by fluorine atoms,

E is an oxygen or sulphur atom, a direct bond, a —C≡C— bond or a $—CR_6{=}CR_7—$ group, $R_6$ being a hydrogen atom or an alkyl group having from 1 to 6 carbon atoms, and $R_7$ being a hydrogen atom, an alkyl group having from 1 to 6 carbon atoms, or a halogen atom,

$R_4$ is a free hydroxy group, which may be replaced by tetrahydropyranyloxy, tetrahydrofuranyloxy, α-ethoxyethoxy, trimethylsilyloxy, dimethyl-tert.-butylsilyloxy, acetoxy, propionyloxy, butyryloxy or benzoyloxy,

$R_5$ is a hydrogen atom, a $C_{1-6}$-alkyl, a halogen-substituted $C_{1-6}$-alkyl, a $C_{1-6}$-cycloalkyl, a phenyl or a 5- or 6-membered heterocyclic group, and, if $R_2$ is a hydrogen atom, the salts thereof with physiologically tolerable bases.

2. Process for the preparation of 9-halo-$\Delta^2$-prostane derivatives of general formula I, characterized in that, in a manner known *per se,* a double bond is introduced into the 2-position of a compound of general formula II

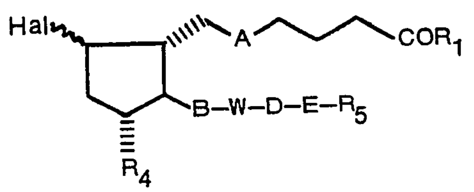

(II),

by reaction with a lithium dialkylamide of general formula III,

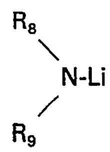

(III),

wherein $R_8$ and $R_9$ are an alkyl group having from 1 to 10 carbon atoms or a cycloalkyl group having from 3 to 6 carbon atoms or a trialkylsilyl group, alkyl being $C_1$—$C_4$-alkyl, and the product is subsequently treated with a phenyl compound of general formula IV,

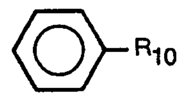

(IV),

wherein $R_{10}$ is the radical

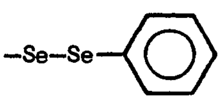 , 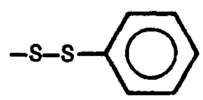 or SeBr

and then, in any order, oxidative elimination is carried out and/or optionally isomers are separated and/or protected hydroxy groups are freed and/or free hydroxy groups are esterified or etherified and/or a free carboxy group is esterified and/or an esterified carboxy group is hydrolysed or a carboxy group is converted into an amide or with a physiologically tolerable base into a salt.

3. Medicament comprising one or more compounds of formula I and customary adjuncts and carriers.